# EUROPEAN PATENT APPLICATION

(11) **EP 1 398 042 A1**
(43) Date of publication of application: **17.03.2004**
(21) Application number: 03255650.8
(22) Date of filing: 10.09.2003
(51) Int. Cl.: A61K 48/00, C12N 15/00

(54) **Transfection of nucleic acid**

(30) Priority: 10.09.2002 US 241312
(71) Applicant: Lipotek, Inc, Taipei, Taiwan (CN)
(72) Inventor: Chang, Fu-Hsiung, Taipai, Taiwan (CN); Lee, Chien-Hsing, Taiwan (CN); Chen, Mingta, Pi-Tou Shrang Chang-Hua County Taiwan (CN)
(74) Representative: Sexton, Jane Helen

(57) **Abstract**

The present invention relates to a substrate for receiving both nucleic acid and eukaryotic cells. The substrate includes a support and a cationic coating thereon prepared from a cationic lipid, a ligand-linked cationic polymer, a mixture of a cationic polymer and a biocompatible biopolymer, a mixture of a cationic polymer and a ligand-linked cationic polymer, or a mixture of a ligand-linked cationic polymer and a biocompatible biopolymer. The cationic coating captures, and facilitates adhesion of, both nucleic acid and eukaryotic cells onto the substrate in a solution. Also disclosed is a method of using a cationic coating to transfect nucleic acid into cells.

## Description

### BACKGROUND

Efficient and facile transfection of nucleic acid is conducive to biological research and clinical applications (e.g., gene therapy, tissue engineering, and in vitro immunization). Among current transfection techniques are cationic lipid-mediated and cationic polymer-mediated methods. Both techniques have certain advantages over others. However, they require calculating the ratio of the number of nitrogen groups in a lipid (or polymer) to the number of the phosphate groups in nucleic acid, mixing the nucleic acid with the lipid (or polymer), and depositing the resultant mixture onto cells. Thus, there is a need for a more convenient method.

### SUMMARY

One aspect of the present invention features a substrate for receiving both nucleic acid and eukaryotic cells. As such, it can promote transfection of the nucleic acid into the cells. This substrate contains a support and a cationic molecule(s) homogenously and non-covalently coated on a surface of the support. The positively charged surface attracts negatively charged nucleic acid. As a result, the nucleic acid concentrates around the cells that adhere to the surface and is transfected into the cells with high efficiency.

The support of the substrate can be formed into various shapes suitable for receiving cells. Examples of the shapes include a tape, a membrane, a thread, a slide, a micro-bead, a micro-particle, a cell culture plate, a multi-well plate, and a bioreactor, all of which can receive cells; and exclude a cylindrical body designed for passing a solution through it and not for receiving cells. Further, the support can be made of metal, polymer, textile, ceramic, plastic, or glass.

The cationic molecule(s) coated on the support can be a cationic lipid, which is optionally associated with a neutral lipid that helps stabilize the coating. Examples of such a neutral lipid include cholesterol, phosphotidylcholine, and phosphotidylethanolamine. A ligand can be linked to either the cationic lipid or the neutral lipid. A cationic lipid (neutral lipid) can be in association with a ligand by having a ligand covalently linked to it or by non-covalent interaction with a ligand-linked neutral lipid (ligand-linked cationic lipid). A ligand is a molecule that binds to a specific type of cell or a molecule that binds to a cell-binding molecule or molecule complex. Examples of the former include a cell adhesion molecule and an antibody against a cell surface marker. An example of the latter is avidin, which can bind to a biotinylated antibody against a cell surface marker. Thus, a ligand-linked cationic lipid or neutral lipid can promote adhesion and transfection.

The cationic molecule(s) of the coated support can also be a cationic polymer (i.e., ligand free), or a ligand-linked cationic polymer, or both. Examples of a cationic polymer include polyethylenimine, polypropyleneimine, poly(amidoamine), and an imidazole-containing polypeptide. Such a cationic polymer can be mixed with a ligand-linked cationic polymer before being coated on the support. Either the cationic polymer or the ligand-linked cationic polymer can also be mixed with a biocompatible biopolymer, such as collagen, gelatin, and chitosan. A biocompatible biopolymer attenuates any toxicity of a cationic polymer, facilitates affixation of the cationic polymer to the support, and provides a favorable environment for cells to grow.

The cationic coating serves to attract nucleic acid to be transfected and is free of any nucleic acid of interest, i.e., it is homogenous. As described above, the cationic coating can contain a neutral lipid (if it is a lipid coating), a biocompatible biopolymer (if it is a polymer coating), or a ligand.

In another aspect, the invention features a method of using one of the substrates described above, i.e., contacting such a substrate with nucleic acid and eukaryotic cells (e.g., mammalian cells), such that both the nucleic acid and the cells adhere onto the cationic coating, resulting in high-efficiency transfection. The cationic coating can be contacted or mixed first with the nucleic acid in vitro, the substrate can further contains a biocompatible biopolymer, and then with the cells in vivo for in vivo transfecting. Alternatively, the cationic coating can be contacted with both the nucleic acid the cells in vitro for in vitro transfection. The support has been successfully used for triple gene transfection and screening the inhibitory efficacy of short interference RNA (siRNA).

Researchers and clinicians can carry out transfection by simply contacting nucleic acid and cells with the cationic coating of the substrate of the invention without the need to prepare a nucleic acid-lipid or nucleic acid-polymer complex as required by the conventional methods. It was unexpected that this less tedious method provides high-efficiency transfection. Furthermore, the substrate and method of the invention are ready to be used with any eukaryotic cell or nucleic acid and can be employed in the fields of functional genomics, high throughput screening, tissue engineering, and scale-up cell culturing.

Other advantages, features, and objects of the invention will be apparent from the detailed description and the claims below.

### DETAILED DESCRIPTION

The present invention relates to a substrate that contains a support and a cationic molecule coated on the support, the cationic molecule being a cationic lipid (ligand-free or ligand-linked), a cationic polymer, or a ligand-linked cationic polymer. The substrate can be prepared by simply dissolving a cationic molecule in a suitable solution, applying the solution onto a surface of the support, and drying the solvent. The resultant substrate can effectively capture nucleic acid, such as an oligonucleotide (see Example 1 below), which, when transfected into cells as an antisense sequence, blocks the expression of an existing protein. The substrate can also effectively capture an expression vector (see Examples 2-8 below.), which, when transfected into cells, enables the cells to produce an exogenous protein.

The support can be in various shapes for different applications. For example, a tape-shaped support can be used in repair of a bone fracture. More specifically, one can adhere mesenchymal cells (progenitors of bone tissue) on the cationic coating of a tape-shaped support and transfect them with an expression vector encoding a factor important for bone development, such as Transforming Growth Factor β (TGF-β) or Bone Morphogenic Protein (BMP). See, e.g., Sikavitsas VI. et al., Biomaterials. 2001 Oct;22(19):2581-93. The coated support, along with the transfected cells, can then be wrapped around or inserted into a bone fracture. Upon expression of the growth factor, the cells develop into bone tissue resulting in healing the bone fracture. Similarly, one can stitch a wound with a thread-shaped support containing a vector encoding a suitable growth factor. In this application, it is not required to adhere cells onto the support prior to stitching. This is because one can transfect cells in the wound tissue and rely on the growth factor produced by the transfected cells to heal the wound. A support in the shape of a slide or a membrane (e.g., cellulose, PVDF, and polycarbonate), on the other hand, can be used to monitor cells transfected with various genes. For example, a cationic molecule can be coated on a slide or membrane in such a way to produce a micropatterned support that contains a large number of separate coated areas. Such a micropatterned support can be used for parallel processing of a large number of cells or nucleic acids. A support in the shape of multi-well plate can be used in a similar manner. In addition, a support in the shape of a bioreactor can be used to transfect and culture cells for large-scale production of recombinant proteins. As an example, the inner surface of the bioreactor is coated with a cationic molecule. The cells can thus be both transfected with an expression vector and cultured in the bioreactor to produce a recombinant protein. Further, the support in the shape of a micro-particle support can be used in the particle bombardment transfection method, in which nucleic acid-coated micro-particles are fired into plant or animal cells, to improve the transfection efficiency.

The support can be made of various materials. For example, a metal support with a cationic coating can be used in the electroporation-transfection method. As nucleic acid concentrates around cells adhered to the metal support, more nucleic acid, upon an electric shock, can be transfected into the cells. In other words, the coating can improve the transfection efficiency of electroporation. A support made of either a metal or a biodegradable polymer can be used for tissue engineering, such as bone reconstruction. Such a support can be modeled after the structure of a bone, e.g., a column with laminar scaffolds in its wall. The column is coated with a cationic molecule for receiving mesenchymal cells and a vector encoding TGF-β or BMP. Upon transfection of TGF-β or BMP into the mesenchymal cells, the transfected cells on the column can then be cultured in vitro or implanted in a patient to convert the column to a bone.

The cationic coating can be formed into a film (i.e., a thin membranous covering) or a foam (i.e., a light frothy mass of fine bubbles) on a support. It can also be tethered to a support via suitable molecules (such as collagen, gelatin, and chitosan) that have been deposited onto the support. See Example 6 below. As mentioned above, the coating can also be micropatterned onto a support. More specifically, a surface of the support can be first patterned with extracellular proteins, such as fibronectin or laminin, using microcontact printing as described in prior art. See, e.g., Kam L. et al. J Biomed Mater Res. 55: 487-95 (2001). The resultant micropatterned surface contains a grid-like array of protein lines. The proteins direct the formation of cationic coating only on unprinted regions of the support. The cationic coating in each of the regions is separated from each other. A support, e.g., a slide, can contain a large number of such regions. It can thus provide a high-throughput system for parallel processing of a large number of cells or nucleic acids.

The cationic molecule can be a cationic lipid, e.g., 1,2 bis(oleoyloxy)-3-(trimethylammonio)propane, didodecyldimethylammonium bromide, dimyristoyloxypropyl-3-dimethylhydroxyethyl ammonium bromide, 1,3-di-oleoyloxy-2-(6-carboxy-spermyl)-propylamid; N-[1,-(2,3-dioleoyloxy) propyl]-N, N, N-trimethylammoniumchloride, doctadecylamidoglycyl-spermine, 3.beta.[N-(N',N'-dimethylaminoethane) carbamoyl] cholesterol, 3beta-[N-(N',N',N'-trimethylaminoethane)carbamoyl] cholesterol, 1,2-dioleoyl-3-(4' trimethylammonio) butanoyl-sn-glycerol, 2'-(1",2"-dioleoyloxypropyldimethyl-ammonium bromide)-N-ethyl-6-amidospermine tetra trifluoroacetic acid, dipalmitoyl phosphatidyesthanolamidospermine, and 1-[2-(9-(Z)-octadecenoyloxy)ethyl]-2-(8-(Z)-heptadecenyl)-3-(hydroxyethyl)imidazolinium chloride. These lipids can be synthesized using well known procedures. See, e.g., Bhattacharya S. et al. Proc. Indian Acad. Sci. Vol. 114, No. 3, June 2002, pp 197-201. A cationic lipid can be mixed with a neutral lipid before being coated on a support. The neutral lipid stabilizes the cationic lipid coating and thereby increases the transfection efficiency. Examples of a suitable neutral lipid for the present invention include phosphotidylethanolamine, cholesterol, and phosphotidylcholine. The amount of the neutral lipid can range from 0.0001 % to 60% (e.g., 30%) of the total lipids.

The cationic molecule can also be a cationic polymer. The molecular weights of suitable cationic polymers can range from 700 to 800,000 Daltons. In one example, a cationic polymer is co-coated with a biocompatible biopolymer, such as collagen, gelatin, and chitosan. The biocompatible biopolymer can be dissolved in water and filtered through a membrane (e.g., 0.45-micrometer filter membrane). The cationic polymer is then dissolved in the filtrate to form a coating solution, preferably with a concentration ranging from 0.1 nanogram/ml to 10 miligram/ml. The concentration of biocompatible biopolymer may be in the range of from 0.1 nanogram/ml to 10 miligram/ml. For the coated surface, the final concentration of cationic polymer on the surface of the support may be in the range of from 0.1 nanogram/cm² to 10 miligram/cm² (e.g. 2 microgram/cm²), whereas the final concentration of the biocompatible biopolymer on the surface of the support may be in the range of from 0.1 nanogram/cm² to 10 miligram/cm² (e.g. 10 nanogram/cm²). According to the invention, the biocompatible biopolymer within such a concentration range facilitates affixation of the cationic polymer to the support, and provides a more favorable environment for cells to grow, thereby the transfection ability of the cationic polymer coated-support is enhanced. The support has been successfully used for triple gene transfection and screening the inhibitory efficacy of short interference RNA (siRNA).

The cationic molecule on a substrate of the invention can be covalently bonded to a ligand. The ligand either binds to a specific type of cells or binds to a molecule (or molecule complex) that binds to a specific type of cells. Examples of a ligand include cell adhesion molecule, antibody, avidin, biotin, folate, carbohydrate, hormone, drug, protein A and protein G. These molecules can capture a specific type of cells from a population of different types of cells and keep them on the substrate. For example, a cationic molecule can be covalently bonded to avidin for binding to the biotin moiety of a biotinylated antibody against a cell-surface marker of lymphocytes. The avidin can thus selectively capture the lymphocytes that have been labeled with the biotinylated antibody. Such a substrate can be used to capture and transfect suspension cells, such as lymphocytes, that normally do not adhere to a support and are difficult to be transfected by conventional methods. Note that one can enrich captured and transfected suspension cells by washing away the nonspecific cells. This one-step transfecting-enriching approach is useful for ex vivo gene therapy in which in vitro transfected cells are enriched and re-introduced into a patient. A ligand-containing substrate can also be used in tissue engineering. For example, one can add an expression vector encoding TGF-β or BMP on a substrate containing an antibody against a surface marker of mesenchymal cells. After the substrate is implanted in a patent, the antibody recruits mesenchymal cells to the substrate, where the cells are transfected with the vectors and develop into bone tissue. Methods for covalently linking a ligand to a cationic molecule are well known in the art. See, e.g., Merdan T. et al, Adv Drug Deliv Rev. 54(5):715 (2002). A ligand can also be linked to the neutral lipid mentioned above. For this purpose, the neutral lipid can be present in an amount of from 0.001 % to 60% (e.g., 10%) by weight of total lipids.

Alternatively, one can include in the cationic coating a molecule that facilitates the entry of nucleic acid into the cells. Examples of such a molecule include a fragment of HIV-tat protein (TAT) and a peptide sequence of hemagglutinin (HA). TAT can bind to and cross cell membranes; HA can aid membrane fusion and the escape from lysosome to cytosol. Thus a TAT- or HA-linked cationic lipid assists nucleic acid in crossing cell membranes and escaping from lysosomes. Also within the scope of this invention are a support coated with such a cationic lipid and a method of using it in transfection.

When using a cationic molecule-coated support to transfect cells, both nucleic acid and cells are incubated with the substrate for a period of time (e.g., 24 hr). Preferably, nucleic acid is first contacted with the cationic molecule, or nucleic acid and cells can be simultaneously contacted with the cationic molecule.

The substrate of the invention is designed to attract both nucleic acid and cells onto a cationic coating, thereby promoting transfection of the former into the latter. However, it can also be used in applications in which no specific nucleic acids are involved. For example, one can make a micropatterned coating with regions containing different ligands against different types of cells and place those cells, together with carrier nucleic acid, onto the coating to form a cell array. One can identify cell targets of a compound by monitoring changes of various cells upon contacting with the compound.

A number of embodiments of the invention have been described. Nevertheless, it will be understood that various modifications may be made without departing from the spirit and scope of the invention. Accordingly, other embodiments are within the scope of the following claims.

The specific examples below are to be construed as merely illustrative, and not limitative of the remainder of the disclosure in any way whatsoever. Without further elaboration, it is believed that one skilled in the art can, based on the description herein, utilize the present invention to its fullest extent. All publications cited herein are hereby incorporated by reference in their entirety.

### Example 1

Standard 96-well plates were coated with 1,2 bis(oleoyloxy)-3-(trimethylammonio)propane (DOTAP; purchased from Avanti, Alabaster, AL, U.S.A.), a cationic lipid. More specifically, 50 microliter aliquots of 2.5 nM, 5 nM, 10 nM, 40 nM, 60 nM, and 80 nM DOTAP methanolic solutions were added to the wells of six sets of the 96-well plates respectively. The plates were then dried overnight in a vacuum-drier and stored in a dessicator until use.

In one experiment, 100 microliters of a complete medium (i.e., RPMI medium containing 10% fetal calf serum, 50 units ml⁻¹ penicillin, and 50 micrograms ml-¹ streptomycin) was added to each well of the coated plates. The coated surfaces and the overlaying medium were then examined under a microscope at 6-hr intervals for 24 hr. The surfaces showed no difference throughout the 24 hr duration for all plates. Also, no liposome-like particles were found either on the coated surfaces or in the medium.

In another experiment, 0.1 microgram of fluorescent-labeled oligonucleotide was added to each well of the coated plates containing 100 microliters of the above-mentioned medium. The plates were then observed under a fluorescent microscope. The fluorescence was detected only on the coated surfaces but not in the medium. This result indicates that the plates coated with DOTAP effectively capture nucleic acid.

### Example 2

The coated plates prepared in Example 1 were used to transfect HeLa cells with an expression plasmid encoding Enhanced Green Fluorescent Protein (EGFP; purchased from Clontech, Inc., Palo Alto, CA, U.S.A.). The EGFP plasmid was purified using the Plasmid Maxi-prep kit of Qiagen (Valencia, CA, U.S.A.), and exhibited an A260/A280 ratio greater than 1.7. Before transfection, 1 x 10⁶ HeLa cells were harvested and resuspended in 5 milliliters of the complete medium described in Example 1. 1 micrograms of the EGFP plasmid was mixed with the cells. The mixture was subsequently placed into the coated plates (about 5,000 cells per well). After the cells had grown on the plates for 24 hr, pictures were taken under a fluorescent microscope. Fluorescent microscopic surface views of the cells revealed that more than 80% of the total HeLa cells expressed high levels of green fluorescent protein (GFP). Furthermore, the cells spread unexpectedly well. This is in contrast to conventional lipid-mediated transfection methods, where cells do not spread well.

### Example 3

Standard 96-well plates were coated with polyethylenimine (PEI), a cationic polymer. More specifically, PEI (MW 25,000 Daltons) was purchased from Sigma-Aldrich (St. Louis, MO, U.S.A.) and dissolved in deionized water to reach a concentration of 10 mg/ml. Each PEI solution was passed through a 0.45-micrometer filter membrane. This filtrate was used as the PEI stock solution and stored at 4 °C. To coat the plates, the stock solution was diluted with sterile deionized water to 20 microgram/ml and a 10-microliter aliquot was added to each well of 96-well plates. The plates were dried and stored as described in Example 1.

To conduct transfection, HeLa cells were mixed with the EGFP plasmid in the complete medium as described in Example 1 and placed onto the PEI-coated plates. 24 hours later, the cells were examined and taken pictures under a fluorescent microscope. Fluorescent microscopic views indicated that nearly 60% of the cells were transfected on all plates, even though most of the cells assumed a rounded and retracted shape, a phenotype of poor cell attachment or cytotoxicity. The PEI density was greater than 0.3 microgram/cm² in this experiment. It was found, unexpectedly, that a surface with a PEI density lower than 0.3 microgram/cm² afforded poor transfection efficacy.

### Example 4

Standard 96-well plates were coated with a PEI a solution containing gelatin and used to transfect HeLa cells with the EGFP plasmid.

More specifically, gelatin was dissolved in deionized water by gentle swirling in a 60°C water bath to form 2% (w/v) gelatin solution. This solution was then slowly cooled down to room temperature and passed through a 0.45-micrometer filter membrane. The filtrate was used as the gelatin stock and was stored at 4°C. This stock solution and the above-mentioned PEI stock solution were mixed and diluted with sterile deionized water so that the final concentration of PEI and gelatin were 20 microgram/ml and 0.02% (w/v), respectively. 50 microliters of the PEI/gelatin solution was added to each well of the plates. The plates were then dried and stored in a dessicator.

HeLa cells were transfected with the EGFP plasmid and cultured using the PEI/gelatin-coated plates, and then examined as described in Example 2. Fluorescent microscopic surface views of the cells revealed that about 80% of the total HeLa cells expressed high levels of GFP. Microscopic surface views also revealed more cells per field on the PEI/gelatin-coated plates than on the PEI-coated plates. Also, the cells grown on the PEI/gelatin coated plates spread out better than the cells grown on the PEI-coated plates (Example 3).

### Example 5

The coated 96-well plates prepared in Example 4 were used to transfect human hepatoma NTU-B W cells, which are notoriously difficult to be transfected using conventional lipid- or polymer-mediated methods.

The cells were transfected and cultured on PEI/gelatin-coated plates, and then examined as described in Example 4. Fluorescent microscopic surface views indicated that about 50% of the total cells expressed high levels of GFP.

### Example 6

Glass slides were tethered with DOTAP or PEI and used to transfect HeLa cells.

More specifically, borosilicate glass slides (VWR Scientific, Media, PA, U.S.A.) were cleaned with Linbro 7X detergent (ICN Biomedicals, Inc., Aurora, OH), diluted 1:3 (v/v) in deionized water, and baked at 450°C for 4 hr. The slides were then printed with gelatin or chitosan by microcontact printing using polydimethylsiloxane (PDMS; Sylgard 184; Dow Corning, Midland, MI, U.S.A.) elastomer stamps as described in prior art. See, e.g., Kam L. et al., J Biomed Mater Res. 55: 487-495 (2001). One milliliter of 10 nM DOTAP solution and 20 microgram/ml PEI solutions were respectively overlaid onto two set of slides for one minute. The slides were then rinsed three times with sterile deionized water and three times with Dulbecco's modified Eagle's medium (DMEM; Gibco BRL, Great Island, NY, U.S.A.) before use.

HeLa cells were mixed with the EGFP plasmid and seeded onto slides that were placed in 100 mm cell culture dishes containing the above-mentioned complete medium. 24 hours later, the cells were observed under a fluorescent microscope and 80% of the cells were found to express GFP.

### Example 7

The dose effect of gelatin on the cell transfection was determined.

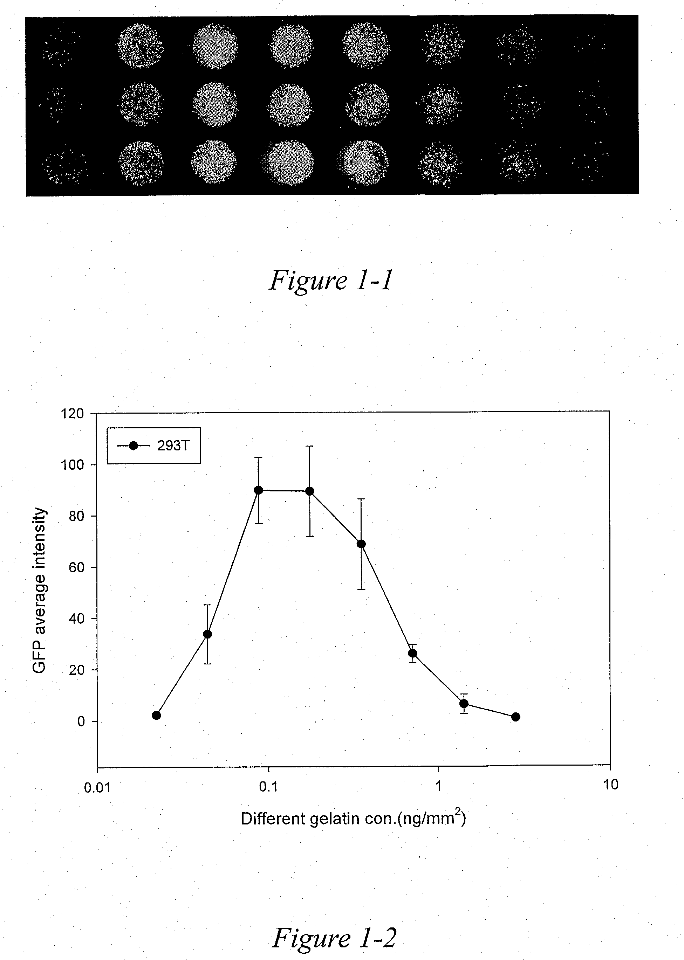

A glass slide with mini-wells from Grace Bio-Labs (Bend, OR, U.S.A.) was coated with 2 microgram/cm² of PEI in the presence of gelatin, ranging from 0:02-2.8 nanogram/mm². Afterward, 1 microgram of EGFP plasmid and 293T cells were applied onto the coated wells. The fluorescent image was taken by a Zeiss dissecting microscope equipped with the cooled CCD (see Figure 1-1). The levels of GFP average intensity at different gelatin concentrations were given in Figure 1-2. It showed that nearly 80% of 293T cells were successfully transfected at the optimal concentration ranging from 0.08 - 0.35 nanogram/mm² on the coated surface. Similar trend has been found when DNA was contacted or mixed first with PEI, further with gelatin, before adding 293T or HeLa cells to the wells.

### Example 8

The dose effect of PEI on the cell trasfection was determined.

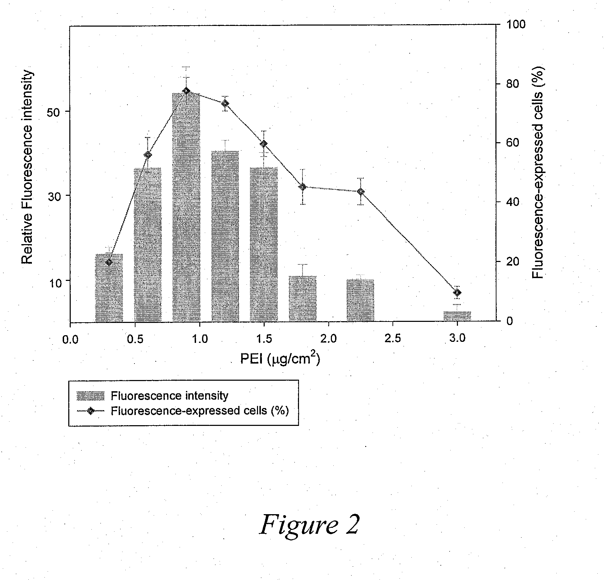

A glass slide with mini-wells from Grace Bio-Labs (Bend, OR, U.S.A.) was coated with 10 nanogram/cm² of gelatin with different amount of PEI, ranging from 0.3-3 microgram/cm². Afterward, 1 microgram of EGFP plasmid and 293T cells were applied onto the coated wells. The fluorescent image was taken by a Zeiss dissecting microscope equipped with the cooled CCD. The levels of relative fluorescence intensity and the percentages of fluorescence-expressed cells at different PEI concentrations were given in Figure 2. It showed that nearly 80% of 293T cells were successfully transfected of the optimal concentration at 1 microgram/cm² on the coated surface. Similar trend has been found when DNA was contacted or mixed first with PEI, further with gelatin, before adding 293T or HeLa cells to the wells.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. Thus, other embodiments are also within the scope of the following claims.

## Claims

1. A substrate for receiving both nucleic acid and eukaryotic cells, the substrate comprising:
(a) a support; and
(b) a cationic molecule;
wherein the cationic molecule captures, and thereby facilitates adhesion, of both nucleic acid and eukaryotic cells.

2. A substrate according to claim 1, wherein the cationic molecule is a cationic lipid.

3. A substrate according to claim 2, further comprising a neutral lipid in association with the cationic lipid.

4. A substrate according to claim 3, wherein the neutral lipid is phosphotidylethanolamine, phosphotidylchorine or cholesterol.

5. A substrate according to claim 3 or 4, further comprising a ligand, said ligand being associated with the cationic lipid or the neutral lipid.

6. A substrate according to claim 1, wherein the cationic molecule is a cationic polymer, a ligand-linked cationic polymer or a mixture thereof.

7. A substrate according to claim 6, further comprising a biocompatible biopolymer.

8. A substrate according to claim 6, wherein the final concentration of the cationic polymer on the surface of the support is from 0.1 nanogram/cm² to 10 miligram/cm².

9. A substrate according to claim 7, wherein the final concentration of the biocompatible biopolymer on the surface of the support is from 0.1 nanogram/cm² to 10 miligram/cm².

10. A substrate according to any one of the preceding claims, wherein the cationic molecule(s) are homogenously and non-covalently disposed on a surface of the support.

11. A substrate according to any one of the preceding claims, wherein the support is metal or ceramic.

12. A substrate according to any one of the preceding claims, wherein the cells are mammalian cells.

13. A substrate according to any one of the preceding claims, wherein the support is in the form of a thread or tape.

14. A method of introducing nucleic acid into eukaryotic cells, the method comprising:
(a) providing a substrate according to any one of the preceding claims;
(b) contacting the cationic molecule with nucleic acid and eukaryotic cells, whereby both the nucleic acid and the eukaryotic cells adhere to the cationic molecule on the substrate.

15. A method according to claim 14, wherein the contacting step (b) is performed by contacting the cationic molecule with the nucleic acid and the cells *in vitro*, or by first contacting the cationic molecule with the nucleic acid *in vitro* and then with the cells *in vitro*.

16. A method according to claim 14, wherein the contacting step (b) is performed by first contacting the cationic polymer with the nucleic acid, further with the biocompatible biopolymer and then with the cells *in vitro.*

17. A substrate according to any one of claims 1 to 13 for use in a method of treatment of the human or animal body by therapy or diagnosis.

18. Use of a substrate according to any one of claims 1 to 13 for the manufacture of a medicament for introducing nucleic acid into eukaryotic cells, by contacting the substrate with nucleic acid and eukaryotic cells whereby both the nucleic acid and the eukaryotic cells adhere to the cationic molecule(s) on the substrate.

19. Use according to claim 18, wherein the contacting step is performed by first contacting the cationic molecule with the nucleic acid in vitro and then with the cells *in vivo*.

20. Use according to claim 18, wherein the contacting step is performed by first contacting the cationic polymer with the nucleic acid in vitro, further with the biocompatible polymer and then with the cells *in vivo*.
